# EUROPEAN PATENT APPLICATION

(11) **EP 4 083 226 A1**
(43) Date of publication of application: **02.11.2022**
(21) Application number: 20905893.2
(22) Date of filing: 01.12.2020
(51) Int. Cl.: C12Q 1/6837, C12Q 1/6827

(54) **TAILORED GENE CHIP FOR GENETIC TEST AND FABRICATION METHOD THEREFOR**

(30) Priority: 27.12.2019 KR 20190175987
(71) Applicant: Clinomics Inc., Ulsan 44919 (KR)
(72) Inventor: CHO, Yun Sung, Yongin-si Gyeonggi-do 16953 (KR); JUN, Je Hoon, Suwon-si Gyeonggi-do 16239 (KR); BHAK, Jong Hwa, Ulju-gun Ulsan 44936 (KR); KIM, Byung Chul, Suwon-si Gyeonggi-do 16713 (KR)
(74) Representative: Altmann Stößel Dick Patentanwälte PartG mbB
(86) International application number: PCT/KR2020/017383
(87) International publication number: WO 2021/132920

(57) **Abstract**

The present invention relates to a method for fabrication of a tailored gene chip for a genetic test and, more specifically, to a method for designing a tailored chip for accuracy improvement in a genetic test, that is, for determining which markers are included upon the fabrication of a tailored gene chip. The tailored gene chip according to the present invention can acquire more accurate data, compared to the use of commercialized gene chips, and thus is advantageous for a gene test. In addition, selection can be made of more accurate markers even from a lower number of markers since a marker selection method used for configuring the tailored gene chip is carried out in consideration of a target ethnic group.

## Description

### [Technical Field]

The present invention relates to a tailored gene chip for genetic test, and more particularly, to a tailored gene chip designed to improve the accuracy of genetic test.

### [Background Art]

In recent years, genetic mutation markers related to diseases and phenotypes have been revealed by genome sequence decoding and disease studies. If there is a mutation of such a revealed gene, the possibility of developing a disease increases, so it is used as a marker for genetic test and is used for disease prediction.

Genetic test is a test method for genes contained in chromosomes and refers to a test method for diagnosing genetic diseases, some tumors, mutations, and chromosomal abnormalities. There are many methods for analyzing DNA at the molecular level, such as polymerase chain reaction (PCR), gene sequencing, and gene chip. Among them, gene chips are easy to use and have many commercially available types, and they have the advantage that they do not consume much time and money even if the analysis for the samples of many people is performed.

However, commercialized gene chips are configured to be used for various diseases and phenotypes in various races. For this reason, there is a limit to efficiently using the gene chip in the commercialized specific ethnic group and specific disease/phenotype. It is not suitable for genetic test that identifies multiple DNA markers because in many cases, the target genetic marker is not included, or the results of the marker cannot be viewed due to problems during the experiment or the bias of the samples even if the marker is present. Therefore, in order to efficiently utilize the gene chip, it is essential to construct a new gene chip in which the desired gene and mutation site is planted. Even when the result of the corresponding marker cannot be confirmed due to a problem during the experiment, there is a need for a method capable of correcting it.

### [Disclosure]

### [Technical Problem]

Accordingly, the present inventors completed the present invention by deriving conditions for selecting closely related markers to increase the prediction accuracy of the target marker while researching a method for increasing the accuracy of genetic test using a gene chip.

Therefore, an object of the present invention is to provide a tailored gene chip with improved accuracy of genetic test, the chip including a target marker; and a nearby marker having a linkage disequilibrium relationship with the target marker.

Another object of the present invention is to provide a method for selecting a nearby marker for improving the accuracy of a genetic test, the method including a step of selecting a target marker and a nearby marker having a linkage disequilibrium relationship with the target marker.

Another object of the present invention is to provide a computer-readable recording medium in which a program for executing the method for selecting a nearby marker on a computer is recorded.

Another object of the present invention is to provide a method for analyzing a single nucleotide polymorphism imputation, the method including steps of: selecting a nearby marker having a linkage disequilibrium relationship with a target marker; and performing the single nucleotide polymorphism imputation using the target marker and the nearby marker.

Another object of the present invention is to provide a computer-readable recording medium recording a program for executing the method for analyzing the single nucleotide polymorphism imputation on a computer.

### [Technical Solution]

In order to achieve the above object, the present invention provides a tailored gene chip with improved accuracy of genetic test, the chip including a target marker; and a nearby marker having a linkage disequilibrium relationship with the target marker.

In addition, the present invention provides a method for selecting a nearby marker for improving the accuracy of a genetic test, the method including a step of selecting a target marker and a nearby marker having a linkage disequilibrium relationship with the target marker.

In addition, the present invention provides a computer-readable recording medium in which a program for executing the method for selecting a nearby marker on a computer is recorded.

In addition, the present invention provides a method for analyzing a single nucleotide polymorphism imputation, the method including steps of: selecting a nearby marker having a linkage disequilibrium relationship with a target marker; and performing the single nucleotide polymorphism imputation using the target marker and the nearby marker.

In addition, the present invention provides a computer-readable recording medium recording a program for executing the method for analyzing the single nucleotide polymorphism imputation on a computer.

### [Advantageous Effects]

The tailored gene chip according to the present invention can acquire data with higher accuracy compared to a use of a commercialized gene chip, which is advantageous for genetic test. In addition, since the method for selecting a marker used to construct the tailored gene chip considers the target ethnic group, it is possible to select a marker with higher accuracy even with a small number of markers.

### [Description of Drawings]

FIG. 1 is a view showing the results of measuring single nucleotide polymorphism imputation accuracy using an Axiom APMRA chip including a disease marker in consideration of the mutation characteristics of Asians and a marker for single nucleotide polymorphism imputation.
FIG. 2 is a view illustrating the results of comparing single nucleotide polymorphism imputation accuracy according to a distance from a target marker.
FIGS. 3 and 4 are views showing results of comparison of single nucleotide polymorphism imputation accuracy according to the frequency of alleles.
FIG. 5 is a view showing the results of comparing the single nucleotide polymorphism imputation accuracy according to the number of alleles of the marker.
FIG. 6 is a view showing the results of a comparison of single nucleotide polymorphism imputation accuracy according to the genotype production rate (calling rate).
FIG. 7 is a view showing the results of comparing the single nucleotide polymorphism imputation accuracy using a commercially available Axiom APMRA chip; and a gene chip including a nearby marker selected according to the present invention.

### [Best Mode of the Invention]

Hereinafter, the present invention is described in detail.

According to an aspect of the present invention, the present invention provides a tailored gene chip with improved accuracy of genetic test, the chip including a target marker; and a nearby marker having a linkage disequilibrium relationship with the target marker.

As used herein, the term "target marker" means a to-be-identified marker through genetic test, and examples thereof include disease-related genetic markers and phenotype-related genetic markers. (i) When the target marker is a disease-related marker, diagnosis or prognosis of a disease can be predicted through identification thereof, and (ii) when the target marker is a phenotype-related marker, the phenotype can be predicted through identification of a genetic marker.

As used herein, the term "linkage disequilibrium (LD)" means that two different alleles appear more closely related than expected value (i.e., theoretical value) due to the non-random linkage of two alleles in different chromosomal regions and is a measure of genetic association. Linkage disequilibrium may be caused by random drift, non-random mating, population structure, etc. in addition to the association of the loci of the two genes.

As used herein, the term "nearby marker" refers to a marker that is genetically closely related to the target marker and may be collected from a mutation database such as dbSNP (Single Nucleotide Polymorphism Database).

As used herein, the term "genetic test" refers to a test method for diagnosing genetic diseases, some tumors, mutations, chromosomal abnormalities, etc., by testing for genes contained in chromosomes. Examples of the genetic test include a polymerase chain reaction, a gene sequencing test, and a gene chip.

As used herein, the term "gene chip (DNA chip)" refers to a biochemical semiconductor made to search tens of thousands to hundreds of thousands of genes at once using hydrogen bonding of nucleotides in adenine-thymine (A-T), guanine-cytosine (G-C) formulas. The gene chip is a new level of an analysis system that can be widely applied to basic research of genes, as well as genetic diagnosis of various diseases, rapid detection of pathogens such as bacteria and viruses, and selection of optimal drugs according to an individual's genetic form.

As used herein, the term "tailored gene chip" is a gene chip specialized for a target marker or a group to be analyzed (e.g., race, species, etc.), and has an advantage in that analysis accuracy is higher than that of a commercialized chip. However, tailored gene chips must be manufactured according to the purpose of the analysis.

In an embodiment of the present invention, the gene chip may be a genetic testing system, a genetic testing device, or a testing device including a gene chip.

In an embodiment of the present invention, the target marker is preferably included in the gene chip two or more times. When the target marker is included in the gene chip two or more times as in the above embodiment, the accuracy of the genetic test can be improved by repeatedly producing genotype information for the same target marker. When the target marker is included in the gene chip less than twice, it is not preferable because data collection is difficult if no results are obtained from the target marker position.

In an embodiment of the present invention, the nearby marker preferably satisfies one or more conditions selected from the group consisting of the following conditions (a) to (d).
- Condition (a): The distance from the target marker is 1 b to 500 Kb
- Condition (b): The frequency of alleles within the to-be-analyzed group is 0.01 to 0.5
- Condition (c): The number of alleles is two (di-allele)
- Condition (d): The calling rate is 50 to 99.99%

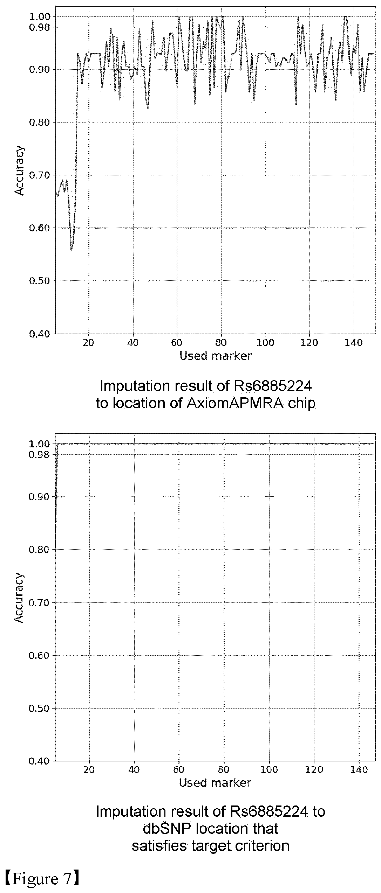

In the example of the present invention, as a result of performing single nucleotide polymorphism imputation of marker rs6885224 using the tailored gene chip according to the present invention, it was confirmed that the accuracy was 99.9% when an average of 17.3 selected nearby markers were used (See FIG. 7). Meanwhile, as a result of performing single nucleotide polymorphism imputation using a gene chip in which markers irrelevant to the selection criteria for nearby markers were arranged, it was confirmed that the accuracy was 93.2% when performed using 150 markers, and increasing the number of markers did not improve the accuracy.

The tailored gene chip according to the present invention (i) includes a target marker repeatedly two or more times, and (ii) includes a nearby marker that satisfies the above conditions so that the accuracy of the genetic test is significantly increased, and data with high accuracy can be obtained even with a small number of nearby markers.

According to another aspect of the present invention, the present invention provides a method for selecting a nearby marker for improving accuracy of a genetic test, the method including a step of selecting a nearby marker having a linkage disequilibrium relationship with a target marker.

In a preferred embodiment of the present invention, the nearby marker is in a close relationship with the target marker and more specifically, is preferably in a linkage disequilibrium relationship.

In an embodiment of the present invention, the nearby marker preferably satisfies one or more conditions selected from the group consisting of the following conditions (a) to (d).
- Condition (a): The distance from the target marker is 1 b to 500 Kb
- Condition (b): The frequency of alleles within the to-be-analyzed group is 0.01 to 0.5
- Condition (c): The number of alleles is two (di-allele)
- Condition (d): The calling rate is 50 to 99.99%

In a preferred embodiment of the present invention, the distance of the nearby marker from the target marker is preferably 1 b to 500 Kb, more preferably 1 b to 300 Kb, and still more preferably 1 b to 250 Kb.

In a preferred embodiment of the present invention, the nearby marker has the frequency of alleles in the to-be-analyzed population of preferably 0.01 to 0.5, and more preferably 0.1 to 0.3.

In a preferred embodiment of the present invention, the number of alleles of the nearby marker is preferably two.

In a preferred embodiment of the present invention, the calling rate of the nearby marker is preferably 50 to 99.99%, more preferably 60 to 99.99%.

The method for selecting a nearby marker according to the present invention can be used to rapidly select a suitable nearby marker according to a target marker or a target ethnic group, and the selected nearby marker can significantly increase the accuracy of a genetic test.

According to another aspect of the present invention, the present invention provides a computer-readable recording medium in which a program for executing the method for selecting a nearby marker is recorded.

As used herein, the term "recording medium" refers to a computer-readable medium as the computer-readable recording medium. The computer-readable recording medium includes all types of recording devices in which data readable by a computer system is stored. Examples of the computer-readable recording medium include a storage medium such as a magnetic storage medium (e.g., a floppy disk, a hard disk, etc.) and an optically readable medium (e.g., a CD, DVD, USB, etc.). In addition, it includes being implemented in the form of a carrier wave (e.g., transmission over the Internet). In addition, the computer-readable recording medium is distributed in a network-connected computer system so that the computer-readable code can be stored and executed in a distributed manner.

According to another aspect of the present invention, the present invention provides a method for analyzing a single nucleotide polymorphism imputation, the method including steps of: selecting a nearby marker having a linkage disequilibrium relationship with a target marker; and performing the single nucleotide polymorphism imputation using the target marker and the nearby marker.

As used herein, the term "single nucleotide polymorphism imputation" is a genetic test, which is a method of inferring the target marker genotype of another subject using the genotype result of another marker (i.e., a nearby marker) in a linkage disequilibrium relationship with high relevance to the target marker.

In an embodiment of the present invention, the target marker is preferably analyzed two or more times for single nucleotide polymorphism imputation.

In an embodiment of the present invention, the nearby marker preferably satisfies one or more conditions selected from the group consisting of the following conditions (a) to (d):
- Condition (a): The distance from the target marker is 1 b to 500 Kb;
- Condition (b): The frequency of alleles within the to-be-analyzed group is 0.01 to 0.5;
- Condition (c): The number of alleles is two (di-allele); and
- Condition (d): The calling rate is 50 to 99.99%.

The analysis method of the single nucleotide polymorphism imputation according to the present invention can acquire data with high accuracy with a smaller number of nearby markers compared to the analysis using nearby markers that are not related to the selection criteria.

According to another aspect of the present invention, the present invention provides a computer-readable recording medium in which a program for executing a method for the single nucleotide polymorphism imputation analysis on a computer is recorded.

### [Modes of the Invention]

Hereinafter, the present invention is described in more detail through examples. These examples are only for illustrating the present invention, and it will be apparent to those of ordinary skill in the art that the scope of the present invention is not to be construed as being limited by these examples.

### Experimental Example 1. Production of gene chip data

In order to produce gene chip data, saliva, gargle, and oral epithelial cells from 7 Koreans produced from whole genome sequence (WGS) data were collected. DNA of the collected samples was extracted using the GeneAll mini kit. Gene chip data of the extracted DNA were produced using Axiom APMRA kit (Asia Precision Medicine Research Array Kit) and GeneTitan equipment. For each sample collected, the process was repeated three times to generate data. The Axiom APMRA chip used for gene chip data production contains more than 750,000 markers, which is composed of about 540,000 markers for a disease marker and single nucleotide polymorphism imputation for precision medicine research that considers Asian mutation characteristics.

### Experimental Example 2. Single nucleotide polymorphism imputation

For a nearby marker for single nucleotide polymorphism imputation of the target gene marker, Korean genotype data on the location of the marker planted in the Axiome APMRA chip were collected and used from dbSNP (Single Nucleotide Polymorphism Database).

The gene chip data produced in Experimental Example 1 was used as input data for single nucleotide polymorphism imputation. In addition, the result of single nucleotide polymorphism imputation was compared with the genotyping result derived from the whole genome sequence (WGS) of the sample to calculate the accuracy.

For single nucleotide polymorphism imputation analysis, software IMPUTE2 (ver2.3.2) was used. In more detail, single nucleotide polymorphism imputation analysis was performed even if there was a genotype result at the corresponding position using the -pgs option of the software IMPUTE2. The -buffer option was used to perform analysis so as to use the information on distant marker. In addition, the default options were used for other options in the software IMPUTE2.

### Example 1. Analysis of single nucleotide polymorphism imputation using Axiom APMRA chip

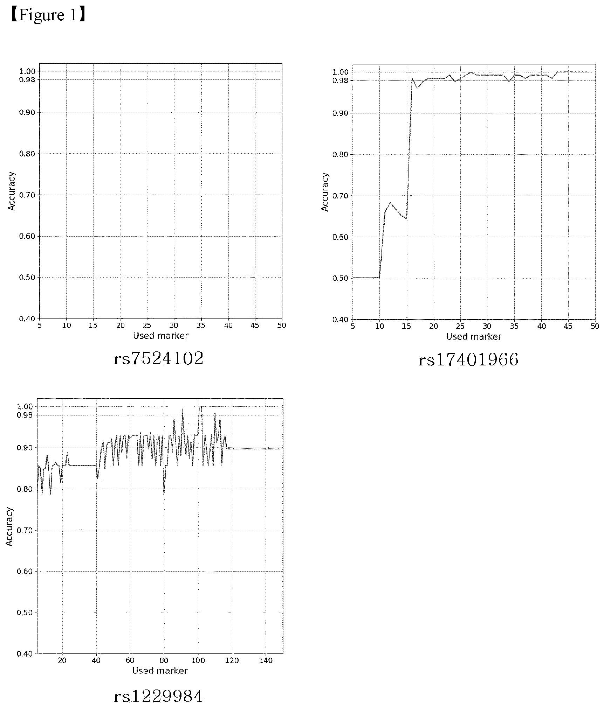

The accuracy of single nucleotide polymorphism imputation for 464 disease-related SNP target markers was measured using the Axiom APMRA chip, which includes a disease marker considering Asian mutation characteristics and a marker for single nucleotide polymorphism imputation. The single nucleotide polymorphism imputation accuracy was analyzed while increasing the nearby markers one by one in the order closest to the target marker, and the minimum number of nearby markers showing an accuracy of 98% or more was confirmed. The results of measuring single nucleotide polymorphism imputation accuracy are shown in FIG. 1.

As shown in FIG. 1, 429 SNP markers including markers rs7524102 and rs17401966 among all 464 SNP markers showed an accuracy of 99.6% or more when an average of 19.9 nearby markers were used. On the other hand, 35 SNP markers (including marker rs1229984) had a low accuracy of 93.2% on average, even though 150 nearby markers were used. As described above, Table 1 shows the SNP markers with low accuracy even when using a large number of nearby markers.

**[Table 1]**

| CHROM | POS | RS-ID | Accuracy when using 10 nearby markers | Accuracy when using 50 nearby markers | Accuracy when using 150 nearby markers |
|---|---|---|---|---|---|
| chr1 | 11856378 | rs1801133 | 0.786 | 0.778 | 0.944 |
| chr1 | 103379918 | rs3753841 | 0.833 | 0.976 | 0.944 |
| chr1 | 161479745 | rs1801274 | 0.921 | 0.937 | 0.976 |
| chr1 | 196679455 | rs10737680 | 0.817 | 0.96 | 0.968 |
| chr1 | 200007432 | rs3790844 | 0.778 | 0.913 | 0.968 |
| chr11 | 2781519 | rs179785 | 0.905 | 0.944 | 0.929 |
| chr11 | 118477367 | rs498872 | 0.786 | 0.929 | 0.929 |
| chr12 | 4368352 | rs10774214 | 0.937 | 0.929 | 0.857 |
| chr12 | 57527283 | rs11172113 | 0.913 | 0.921 | 0.913 |
| chr13 | 42754522 | rs4142110 | 0.698 | 0.905 | 0.857 |
| chr14 | 100133942 | rs2895811 | 0.722 | 0.929 | 0.968 |
| chr15 | 28197037 | rs1800414 | 0.786 | 0.929 | 0.976 |
| chr15 | 78915245 | rs6495309 | 0.913 | 0.968 | 0.944 |
| chr15 | 91512067 | rs2290203 | 0.929 | 0.929 | 0.929 |
| chr16 | 1532463 | rs13336428 | 0.754 | 0.921 | 0.929 |
| chr17 | 800593 | rs12603526 | 0.802 | 0.921 | 0.976 |
| chr17 | 59447369 | rs11653176 | 0.937 | 0.929 | 0.976 |
| chr19 | 7166109 | rs2059807 | 0.857 | 0.881 | 0.929 |
| chr19 | 19379549 | rs58542926 | 0.849 | 0.929 | 0.929 |
| chr2 | 145223620 | rs13382811 | 0.849 | 0.849 | 0.929 |
| chr2 | 198631714 | rs700651 | 0.841 | 0.929 | 0.913 |
| chr21 | 44588757 | rs7278468 | 0.921 | 0.921 | 0.857 |
| chr22 | 37635055 | rs2284038 | 0.952 | 0.857 | 0.929 |
| chr22 | 43500212 | rs5759167 | 0.786 | 0.937 | 0.921 |
| chr5 | 11169945 | rs6885224 | 0.69 | 0.921 | 0.897 |
| chr5 | 59502520 | rs966221 | 0.786 | 0.929 | 0.976 |
| chr5 | 158764177 | rs7709212 | 0.722 | 0.929 | 0.929 |
| chr5 | 168195356 | rs11134527 | 0.857 | 0.921 | 0.944 |
| chr6 | 38365841 | rs9296249 | 0.96 | 0.929 | 0.857 |
| chr6 | 101964914 | rs9390754 | 0.921 | 0.929 | 0.929 |
| chr7 | 19049388 | rs2107595 | 0.968 | 0.96 | 0.976 |
| chr7 | 37746569 | rs2392510 | 0.817 | 0.905 | 0.857 |
| chr7 | 74126034 | rs117026326 | 0.929 | 0.929 | 0.929 |
| chr8 | 11480457 | rs2243407 | 0.857 | 0.929 | 0.976 |
| chr9 | 9261737 | rs4626664 | 0.81 | 0.929 | 0.968 |

The above results indicate that some markers have low accuracy, so it is inappropriate to use the Axiom APMRA chip for genetic test.

### Example 2. Optimization of conditions for selecting nearby markers to increase accuracy of single nucleotide polymorphism imputation

In order to correct the 35 markers with low accuracy identified in Example 1, SNPs (i.e., nearby markers) were selected from dbSNPs under respective different conditions, and the accuracy of single nucleotide polymorphism imputation was compared.

### 2-1. Comparison of single nucleotide polymorphism imputation accuracy according to distance from target marker

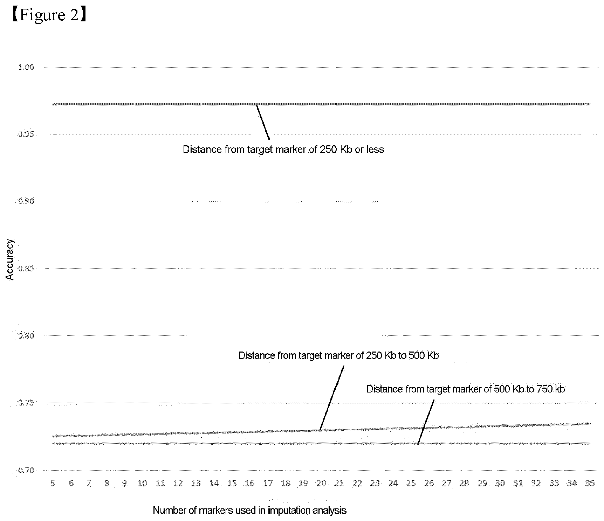

The single nucleotide polymorphism imputation accuracy of the nearby marker was compared according to the distance from the target marker. First, nearby markers were divided into three groups based on distance from the target marker, i.e., (a) less than 250 Kb; (b) 250 Kb or more and less than 500 Kb; and (c) 500 Kb or more and less than 750 Kb. The nearby markers were analyzed in increasing order, one by one, in the order close to the target marker. A comparison result of single nucleotide polymorphism imputation accuracy is shown in FIG. 2.

As shown in FIG. 2, the group using only nearby markers with a distance from the target marker of less than 250 Kb had the highest single nucleotide polymorphism imputation accuracy. On the other hand, it was confirmed that the group using a nearby marker having a distance from the target marker of 250 Kb or more had low single nucleotide polymorphism imputation accuracy.

### 2-2. Comparison of single nucleotide polymorphism imputation accuracy according to allele frequency

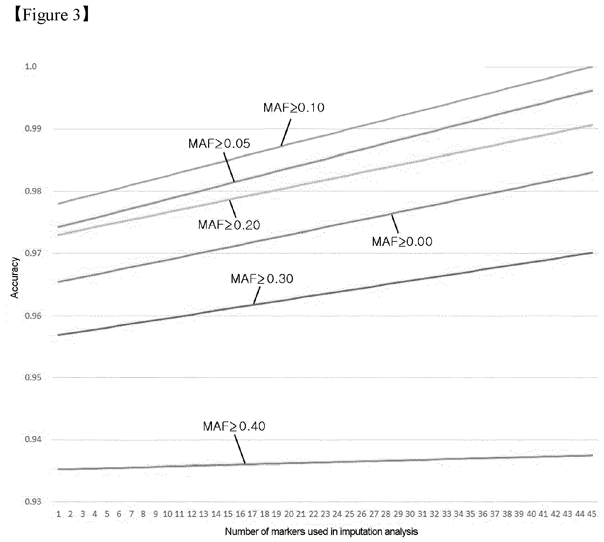

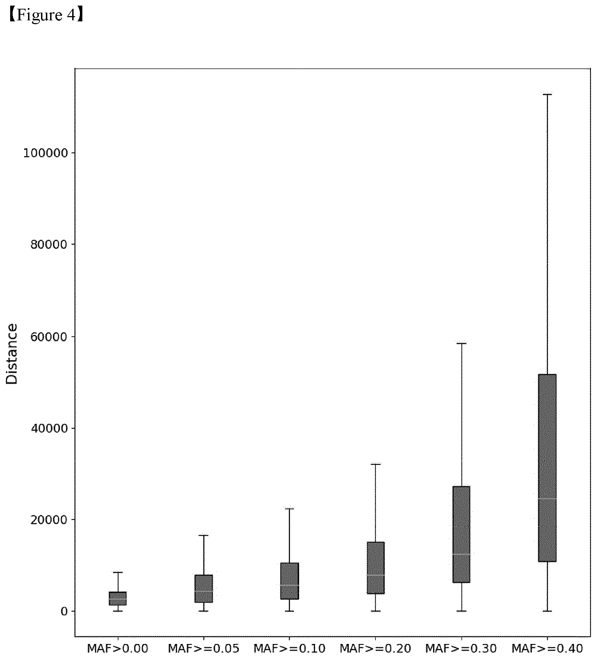

The accuracy of single nucleotide polymorphism imputation according to allele frequency was compared. First, the nearby markers were divided into six groups based on the allele frequency of the to-be-tested population, i.e., (a) 0 or more, (b) 0.05 or more, (c) 0.10 or more, (d) 0.20 or more, (e) 0.30 or more, and (f) 0.40 or greater. The nearby markers were analyzed in increasing order, one by one, in the order close to the target marker. The comparison results of single nucleotide polymorphism imputation accuracy are shown in FIGS. 3 and 4.

As shown in FIG. 3, the single nucleotide polymorphism imputation accuracy was the highest when a nearby marker having an allele frequency of (c) 0.10 or more was used. Next, the single nucleotide polymorphism imputation accuracy of the nearby markers with allele frequencies of (b) 0.05 or more and (d) 0.20 or more was high.

In addition, as shown in FIG. 4, it was confirmed that as the allele frequency increased, the distance between the nearby marker and the target marker increased.

### 2-3. Comparison of single nucleotide polymorphism imputation accuracy according to number of alleles of marker

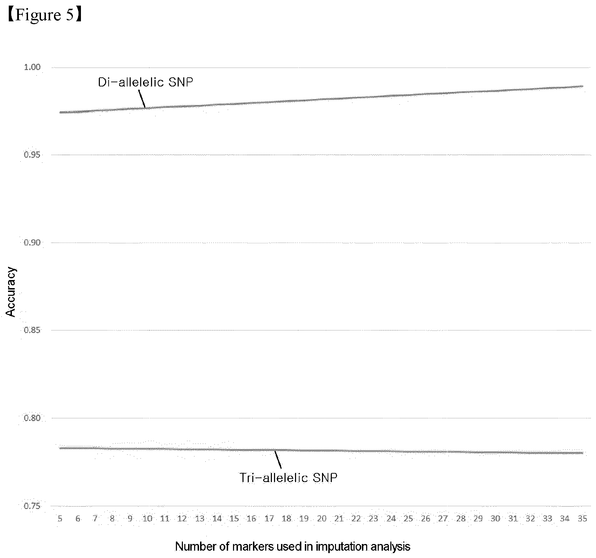

The single nucleotide polymorphism imputation accuracy according to the number of alleles was determined using a tri-allele SNP with three alleles and a di-allele SNP with two alleles. The accuracy was analyzed by increasing the nearby markers one by one in the order closest to the target marker. A comparison result of single nucleotide polymorphism imputation accuracy is shown in FIG. 5.

As shown in FIG. 5, the nearby markers having two alleles had significantly higher single nucleotide polymorphism imputation accuracy compared to the nearby markers having three alleles.

### 2-4. Comparison of single nucleotide polymorphism imputation accuracy according to genotype production rate (Call rate)

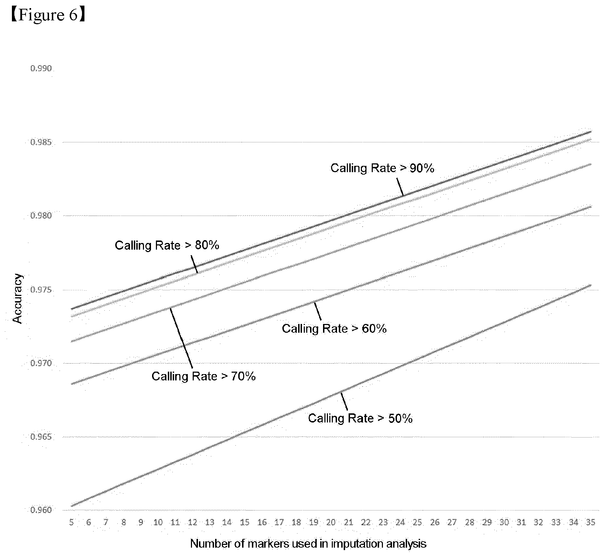

There are regions with a low genotype production rate depending on the to-be-analyzed population group and the genomic data production method. Accordingly, the accuracy of single nucleotide polymorphism imputation was analyzed by using a nearby marker at the same location but varying the genotype production rate of dbSNP. The comparison result of single nucleotide polymorphism imputation accuracy is shown in FIG. 6.

As shown in FIG. 6, it was confirmed that the higher the genotype production rate of the nearby marker, the higher the single nucleotide polymorphism imputation accuracy.

Based on the above results, the conditions for selecting nearby markers to increase the accuracy of single nucleotide polymorphism imputation are as follows.
- Distance from the target marker: less than 250 Kb
- Frequency of alleles: 0.1 or more
- Number of alleles of marker: two (di-allele)
- Calling rate: 90% or more

### Example 3. Analysis of single nucleotide polymorphisms imputation accuracy using nearby markers selected as optimal conditions

Among the markers included in the Axiom APMRA chip, the number of markers corresponding to the conditions for selecting nearby markers of Example 2 was confirmed.

As a result, it was confirmed that the average number of markers satisfying the conditions for selecting nearby markers of Example 2 in the Axiom APMRA chip was 31 for low-accuracy markers and 49 for high-accuracy markers in single nucleotide polymorphism imputation.

Therefore, nearby markers were selected in order to further improve the accuracy of single nucleotide polymorphism imputation. Specifically, among the locations registered in the dbSNP, a nearby marker satisfying the conditions for selecting a nearby marker of Example 2 was confirmed. Through this, 150 or more nearby markers for target marker replacement were selected. Among them, nearby markers related to the marker rs6885224 are shown in Table 2. The accuracy of single nucleotide polymorphism imputation was confirmed using a tailored gene chip including a target marker repeated twice or more together with the selected nearby marker, and the results are shown in FIG. 7.

**[Table 2]**

| RS-ID of target markers | RS-ID of nearby markers | Distance from target marker (Kb) | Frequency of alleles | Type of alleles | Calling rate |
|---|---|---|---|---|---|
| rs6885224 | rs917012 | 84 | 0.88 | C,T | 1.00 |
| rs6885224 | rs16901339 | 344 | 0.28 | T,C | 1.00 |
| rs6885224 | rs13182209 | 561 | 0.31 | C,T | 1.00 |
| rs6885224 | rs6860246 | 1203 | 0.76 | T,C | 1.00 |
| rs6885224 | rs6879413 | 1332 | 0.28 | G,C | 1.00 |
| rs6885224 | rs10078958 | 1707 | 0.41 | T,A | 1.00 |
| rs6885224 | rs10071197 | 1729 | 0.76 | G,T | 1.00 |
| rs6885224 | rs61751836 | 2171 | 0.26 | C,T | 1.00 |
| rs6885224 | rs10513079 | 2352 | 0.26 | T,C | 1.00 |
| rs6885224 | rs7702295 | 2445 | 0.53 | C,T | 1.00 |
| rs6885224 | rs72646806 | 2481 | 0.28 | A,C | 1.00 |
| rs6885224 | rs6861205 | 2580 | 0.31 | A,G | 1.00 |
| rs6885224 | rs7713461 | 2668 | 0.41 | G,A | 1.00 |
| rs6885224 | rs6883143 | 2791 | 0.49 | T,C | 1.00 |
| rs6885224 | rs16901347 | 2828 | 0.24 | T,C | 1.00 |
| rs6885224 | rs13155944 | 2993 | 0.43 | G,A | 1.00 |
| rs6885224 | rs12716080 | 2997 | 0.72 | G,T | 0.99 |
| rs6885224 | rs13362481 | 3046 | 0.41 | C,T | 0.99 |
| rs6885224 | rs61751837 | 3058 | 0.41 | G,T | 1.00 |
| rs6885224 | rs59700924 | 3363 | 0.26 | C,T | 1.00 |
| rs6885224 | rs2057793 | 3727 | 0.54 | C,A | 1.00 |
| rs6885224 | rs6892933 | 3966 | 0.41 | C,G | 0.99 |
| rs6885224 | rs4702790 | 4102 | 0.79 | C,T | 1.00 |
| rs6885224 | rs16901333 | 4179 | 0.26 | C,T | 1.00 |
| rs6885224 | rs12655907 | 5432 | 0.74 | T,A | 0.99 |
| rs6885224 | rs721768 | 5584 | 0.54 | C,T | 1.00 |
| rs6885224 | rs61751852 | 5773 | 0.26 | T,C | 1.00 |
| rs6885224 | rs7703504 | 5961 | 0.41 | T,C | 1.00 |
| rs6885224 | rs7721243 | 6075 | 0.41 | G,A | 1.00 |
| rs6885224 | rs1012092 | 6442 | 0.74 | C,T | 1.00 |
| rs6885224 | rs7715991 | 6816 | 0.88 | C,G | 1.00 |
| rs6885224 | rs79140420 | 6923 | 0.39 | C,T | 0.95 |
| rs6885224 | rs7715051 | 7441 | 0.41 | C,G | 1.00 |
| rs6885224 | rs61751855 | 7728 | 0.25 | A,G | 1.00 |
| rs6885224 | rs1978156 | 8320 | 0.89 | T,C | 1.00 |
| rs6885224 | rs10052776 | 8703 | 0.23 | C,T | 1.00 |
| rs6885224 | rs2023916 | 8708 | 0.88 | C,T | 1.00 |
| rs6885224 | rs7705503 | 8907 | 0.63 | G,C | 1.00 |
| rs6885224 | rs12716081 | 9142 | 0.23 | T,C | 1.00 |
| rs6885224 | rs16901329 | 9242 | 0.25 | C,G | 1.00 |
| rs6885224 | rs13179953 | 9248 | 0.17 | T,C | 1.00 |
| rs6885224 | rs7704256 | 9514 | 0.41 | C,G | 1.00 |
| rs6885224 | rs10062829 | 9549 | 0.12 | T,C | 1.00 |
| rs6885224 | rs2277054 | 9902 | 0.42 | A,G | 1.00 |
| rs6885224 | rs1990003 | 10288 | 0.56 | T,C | 1.00 |
| rs6885224 | rs1978155 | 10784 | 0.57 | C,T | 1.00 |
| rs6885224 | rs61753306 | 10853 | 0.25 | T,G | 1.00 |
| rs6885224 | rs6892303 | 11492 | 0.62 | C,A | 1.00 |
| rs6885224 | rs1990004 | 13673 | 0.56 | C,T | 1.00 |
| rs6885224 | rs1990005 | 14140 | 0.58 | C,G | 1.00 |
| rs6885224 | rs1990006 | 14151 | 0.56 | C,G | 1.00 |
| rs6885224 | rs4702791 | 14283 | 0.25 | G,C | 1.00 |
| rs6885224 | rs2057795 | 15348 | 0.71 | T,C | 1.00 |
| rs6885224 | rs13358276 | 15498 | 0.39 | C,T | 1.00 |
| rs6885224 | rs11953748 | 16018 | 0.24 | G,A | 1.00 |
| rs6885224 | rs72730911 | 16556 | 0.39 | A,T | 1.00 |
| rs6885224 | rs32280 | 16707 | 0.22 | G,A | 1.00 |
| rs6885224 | rs72730910 | 17574 | 0.12 | A,G | 1.00 |
| rs6885224 | rs73051687 | 17590 | 0.2 | T,C | 1.00 |
| rs6885224 | rs32277 | 17864 | 0.16 | G,A | 1.00 |
| rs6885224 | rs10059397 | 18026 | 0.39 | C,G | 1.00 |
| rs6885224 | rs66554550 | 18681 | 0.25 | T,A | 1.00 |
| rs6885224 | rs6877976 | 19045 | 0.39 | G,A | 0.99 |
| rs6885224 | rs62339099 | 19576 | 0.39 | T,C | 1.00 |
| rs6885224 | rs10055024 | 20137 | 0.39 | C,T | 1.00 |
| rs6885224 | rs32275 | 20394 | 0.22 | T,C | 1.00 |
| rs6885224 | rs2023534 | 20710 | 0.16 | C,G | 1.00 |
| rs6885224 | rs10036188 | 20742 | 0.39 | G,A | 1.00 |
| rs6885224 | rs32274 | 21053 | 0.46 | C,A | 1.00 |
| rs6885224 | rs757458 | 21108 | 0.39 | A,C | 1.00 |
| rs6885224 | rs757459 | 21252 | 0.39 | T,C | 1.00 |
| rs6885224 | rs12514212 | 21408 | 0.39 | C,T | 1.00 |
| rs6885224 | rs2400030 | 21527 | 0.39 | C,T | 1.00 |
| rs6885224 | rs2400029 | 21688 | 0.39 | C,T | 1.00 |
| rs6885224 | rs886525 | 21834 | 0.39 | C,A | 1.00 |
| rs6885224 | rs886526 | 21918 | 0.46 | A,C | 1.00 |
| rs6885224 | rs886527 | 22131 | 0.46 | G,A | 1.00 |
| rs6885224 | rs79989660 | 22161 | 0.4 | A,G | 0.99 |
| rs6885224 | rs35081177 | 22185 | 0.11 | A,G | 1.00 |
| rs6885224 | rs1859382 | 22273 | 0.46 | G,C | 1.00 |
| rs6885224 | rs12516262 | 22370 | 0.39 | T,G | 1.00 |
| rs6885224 | rs6881290 | 22451 | 0.46 | T,C | 1.00 |
| rs6885224 | rs6859601 | 22531 | 0.46 | C,T | 1.00 |
| rs6885224 | rs6880938 | 22643 | 0.71 | T,C | 1.00 |
| rs6885224 | rs62339097 | 22673 | 0.25 | A,G | 1.00 |
| rs6885224 | rs62339096 | 22753 | 0.24 | C,T | 1.00 |
| rs6885224 | rs6885587 | 22831 | 0.71 | G,C | 1.00 |
| rs6885224 | rs10041627 | 22863 | 0.71 | T,G | 1.00 |
| rs6885224 | rs10059890 | 23247 | 0.46 | C,T | 1.00 |
| rs6885224 | rs10067858 | 23307 | 0.71 | T,C | 1.00 |
| rs6885224 | rs10067783 | 23404 | 0.71 | T,C | 1.00 |
| rs6885224 | rs10074637 | 23465 | 0.71 | A,C | 1.00 |
| rs6885224 | rs10059740 | 23466 | 0.71 | C,T | 1.00 |
| rs6885224 | rs10059698 | 23494 | 0.71 | C,T | 1.00 |
| rs6885224 | rs10073056 | 23692 | 0.71 | A,C | 1.00 |
| rs6885224 | rs10066098 | 23744 | 0.71 | T,C | 1.00 |
| rs6885224 | rs12516033 | 24079 | 0.71 | G,T | 1.00 |
| rs6885224 | rs57133851 | 24150 | 0.25 | T,C | 1.00 |
| rs6885224 | rs2158445 | 24869 | 0.72 | T,A | 0.95 |
| rs6885224 | rs7713340 | 25332 | 0.42 | A,G | 1.00 |
| rs6885224 | rs34061113 | 25466 | 0.3 | G,A | 1.00 |
| rs6885224 | rs7709436 | 25707 | 0.49 | G,A | 1.00 |
| rs6885224 | rs40291 | 25777 | 0.21 | T,C | 1.00 |
| rs6885224 | rs10053794 | 25933 | 0.9 | G,C | 1.00 |
| rs6885224 | rs62339093 | 26460 | 0.26 | G,T | 1.00 |
| rs6885224 | rs32271 | 26767 | 0.21 | T,C | 1.00 |
| rs6885224 | rs32270 | 27080 | 0.21 | G,T | 1.00 |
| rs6885224 | rs32267 | 27550 | 0.21 | G,T | 1.00 |
| rs6885224 | rs32266 | 28484 | 0.21 | A,G | 1.00 |
| rs6885224 | rs32265 | 29029 | 0.21 | A,G | 1.00 |
| rs6885224 | rs16901308 | 29034 | 0.17 | A,G | 1.00 |
| rs6885224 | rs32264 | 29356 | 0.21 | A,T | 1.00 |
| rs6885224 | rs10044129 | 30587 | 0.18 | C,T | 1.00 |
| rs6885224 | rs730610 | 30894 | 0.73 | A,T | 1.00 |
| rs6885224 | rs27720 | 31916 | 0.14 | A,G | 1.00 |
| rs6885224 | rs6874039 | 32024 | 0.73 | C,T | 1.00 |
| rs6885224 | rs6873671 | 32250 | 0.73 | C,G | 1.00 |
| rs6885224 | rs6873490 | 32507 | 0.73 | G,A | 1.00 |
| rs6885224 | rs2214599 | 33052 | 0.41 | C,T | 1.00 |
| rs6885224 | rs1302802 | 33481 | 0.28 | C,T | 1.00 |
| rs6885224 | rs7736192 | 33643 | 0.73 | A,C | 1.00 |
| rs6885224 | rs7714658 | 33694 | 0.73 | T,C | 1.00 |
| rs6885224 | rs7732347 | 33714 | 0.73 | A,G | 1.00 |
| rs6885224 | rs7732720 | 33781 | 0.11 | G,A | 1.00 |
| rs6885224 | rs7731822 | 34048 | 0.73 | A,G | 1.00 |
| rs6885224 | rs6892380 | 34330 | 0.73 | C,T | 1.00 |
| rs6885224 | rs7713315 | 34737 | 0.1 | C,T | 1.00 |
| rs6885224 | rs10513073 | 35173 | 0.72 | C,T | 1.00 |
| rs6885224 | rs62339082 | 35574 | 0.42 | C,A | 1.00 |
| rs6885224 | rs6881497 | 36040 | 0.72 | C,G | 1.00 |
| rs6885224 | rs6876115 | 36792 | 0.67 | C,G | 1.00 |
| rs6885224 | rs2158444 | 37603 | 0.67 | G,T | 1.00 |
| rs6885224 | rs9942305 | 37760 | 0.1 | C,T | 1.00 |
| rs6885224 | rs10069711 | 37950 | 0.68 | G,T | 1.00 |
| rs6885224 | rs149188423 | 38111 | 0.11 | G,T | 1.00 |
| rs6885224 | rs10069596 | 38112 | 0.68 | G,A | 1.00 |
| rs6885224 | rs62339081 | 38270 | 0.24 | G,A | 1.00 |
| rs6885224 | rs6865035 | 38831 | 0.24 | G,A | 1.00 |
| rs6885224 | rs6864900 | 38888 | 0.24 | G,C | 1.00 |
| rs6885224 | rs153603 | 39885 | 0.19 | T,C | 1.00 |
| rs6885224 | rs13189665 | 40373 | 0.86 | A,G | 1.00 |
| rs6885224 | rs73742130 | 40426 | 0.35 | C,T | 1.00 |
| rs6885224 | rs16901404 | 41987 | 0.1 | T,A | 1.00 |
| rs6885224 | rs11748430 | 42226 | 0.1 | A,G | 1.00 |
| rs6885224 | rs1547940 | 42586 | 0.46 | T,C | 1.00 |
| rs6885224 | rs739957 | 42726 | 0.1 | C,G | 1.00 |
| rs6885224 | rs11133648 | 42754 | 0.37 | T,C | 0.99 |
| rs6885224 | rs2023923 | 43154 | 0.37 | C,A | 1.00 |
| rs6885224 | rs76254229 | 43306 | 0.21 | C,T | 1.00 |
| rs6885224 | rs79655595 | 43332 | 0.33 | T,C | 1.00 |

As shown in FIG. 7, in the case of the marker rs6885224, the accuracy of single nucleotide polymorphism imputation using the gene chip of this example was 99.9% when an average of 17.3 selected nearby markers were used. On the other hand, the single nucleotide polymorphism imputation accuracy of the Axiom APMRA chip in which markers independent of the selection criteria were arranged did not increase even if the number of markers was increased. When 150 markers were used, the accuracy was 93.2%. The above result means that the nearby marker selected according to the conditions of Example 2 can significantly improve the single nucleotide polymorphism imputation accuracy.

In order to improve the accuracy of single nucleotide polymorphism imputation, the present inventors have comprehensively developed a tailored gene chip including (i) a target marker repeatedly included two or more times, and (ii) a nearby marker satisfying the following conditions.
- Distance from the target marker: less than 250 Kb
- Frequency of alleles: 0.1 or more
- Number of alleles of marker: two (di-allele)
- Calling rate: 90% or more

The tailored gene chip according to the present invention has significantly increased the accuracy of single nucleotide polymorphism imputation, and high-accuracy data can be obtained even with a small number of nearby markers.

As above, a specific part of the present invention has been described in detail. It is clear for those of ordinary skill in the art that this specific description is only a preferred embodiment, and the scope of the present invention is not limited thereby. Accordingly, it is intended that the substantial scope of the present invention be defined by the appended claims and their equivalents.

## Claims

1. A tailored gene chip with improved accuracy of a genetic test, the chip comprising:
a target marker; and
a nearby marker having a linkage disequilibrium relationship with the target marker.

2. The gene chip of claim 1, wherein the target marker is included two or more times in the gene chip.

3. The gene chip of claim 1, wherein the nearby marker satisfies one or more conditions selected from the group consisting of
condition (a) in which a distance from the target marker is 1 b to 500 Kb;
condition (b) in which a frequency of alleles is 0.01 to 0.5 in a population to be analyzed;
condition (c) in which the number of alleles is two (Di-allele); and
condition (d) in which a calling rate is 50 to 99.99%.

4. A method for selecting a nearby marker for improving accuracy of a genetic test, the method comprising a step of selecting a nearby marker having a linkage disequilibrium relationship with a target marker.

5. The method of claim 4, wherein the nearby marker satisfies one or more conditions selected from the group consisting of
condition (a) in which a distance from the target marker is 1 b to 500 Kb;
condition (b) in which a frequency of alleles is 0.01 to 0.5 in a population to be analyzed;
condition (c) in which the number of alleles is two (Di-allele); and
condition (d) in which a calling rate is 50 to 99.99%.

6. A computer-readable recording medium recording a program for executing the method for selecting a nearby marker according to claim 4 or 5 on a computer.

7. A method for analyzing a single nucleotide polymorphism imputation, the method comprising steps of:
selecting a nearby marker having a linkage disequilibrium relationship with a target marker; and
performing the single nucleotide polymorphism imputation using the target marker and the nearby marker.

8. The method of claim 7, wherein the single nucleotide polymorphism imputation is to analyze the target marker two or more times.

9. The method of claim 7, wherein the nearby marker satisfies one or more conditions selected from the group consisting of
condition (a) in which a distance from the target marker is 1 b to 500 Kb;
condition (b) in which a frequency of alleles is 0.01 to 0.5 in a population to be analyzed;
condition (c) in which the number of alleles is two (Di-allele); and
condition (d) in which a calling rate is 50 to 99.99%.

10. A computer-readable recording medium recording a program for executing the method for analyzing the single nucleotide polymorphism imputation according to any one of claims 7 to 9 on a computer.
